# EUROPÄISCHE PATENTANMELDUNG

(11) **EP 3 656 411 A1**
(43) Veröffentlichungstag der Anmeldung: **27.05.2020**
(21) Anmeldenummer: 18208218.0
(22) Anmeldetag: 26.11.2018
(51) Int. Cl.: A61M 1/10, A61M 1/12

(54) **BLUTPUMPE ZUM UNTERSTÜTZEN EINER HERZFUNKTION UND VERFAHREN ZUR HERSTELLUNG EINES PUMPENGEHÄUSES EINER BLUTPUMPE**

(71) Anmelder: Berlin Heart GmbH, 12247 Berlin (DE)
(72) Erfinder: WINTERWERBER, Kim, 12357 Berlin (DE)
(74) Vertreter: Pfenning, Meinig & Partner mbB

(57) **Zusammenfassung**

Die vorliegende Anmeldung betrifft eine Blutpumpe (2) zum Unterstützen einer Herzfunktion und ein Verfahren zur Herstellung eines Pumpengehäuses (4) der Blutpumpe (2). Die vorgeschlagene Blutpumpe (2) umfasst ein implantierbares Pumpengehäuse (4). Außerdem umfasst die Blutpumpe (2) einen faseroptischen Sensor (28) mit einer Lichtleitfaser (8). Die Lichtleitfaser (8) ist zumindest teilweise in einem Gehäuseteil (12) des Pumpengehäuses (4) aufgenommen.

## Beschreibung

Die vorliegende Anmeldung liegt auf dem Gebiet der Medizintechnik und insbesondere auf dem Gebiet der implantierbaren Blutpumpen zur Unterstützung einer Herzfunktion. Die Anmeldung betrifft eine Blutpumpe zum Unterstützen einer Herzfunktion. Außerdem betrifft die vorliegende Anmeldung ein Verfahren zur Herstellung eines Pumpengehäuses.

Blutpumpen sind aus dem Stand der Technik bekannt. Diese Blutpumpen können zum Einsatz kommen, wenn eine Herzfunktion eines Patienten unterstützt oder ersetzt werden muss. Gängige Systeme, die hierbei verwendet werden, sind sogenannte VAD (ventricular assist devices). Derartige Herzpumpen können beispielsweise als sog. LVAD (left ventricular assist device), RVAD (right ventricular assist device) oder BiVAD (bi-ventricular assist device) ausgeführt sein. Diese Systeme umfassen neben der Blutpumpe, die im Betrieb in dem Patienten implantiert ist, in der Regel ein außerhalb eines Körpers des Patienten angeordnetes und mit der Blutpumpe über eine perkutane Leitung (Driveline) verbundenes Steuergerät. Die Blutpumpe umfasst in der Regel einen Motor mit einem Stator und einem mit einer Beschaufelung versehenen Rotor. Durch von dem Steuergerät gelieferte elektrische Energie kann der Motor der Blutpumpe angetrieben werden, indem beispielsweise ein Stromfluss in Wicklungen des Stators erzeugt wird, durch den der Rotor mitsamt Beschaufelung zum Fördern von Blut des Patienten in Drehung versetzt wird. In einigen Ausführungen können bekannte Systeme zur Unterstützung einer Herzfunktion einen Blutdrucksensor aufweisen. Zudem kann eine Regeleinrichtung zur Regelung der Blutpumpe vorgesehen sein, die eine Förderleistung der Herzpumpe anhand von Blutdruckwerten, die von dem Blutdrucksensor ermittelt wurden, einstellt. Systeme verwandter Art sind beispielsweise in den Druckschriften EP 3 090 767 A1 und EP 3 108 809 A1 beschrieben.

Es ist eine Aufgabe der vorliegenden Anmeldung eine verbesserte Blutpumpe vorzuschlagen. Insbesondere ist es eine Aufgabe der vorliegenden Anmeldung, eine Blutpumpe vorzuschlagen, die bei einer kompakten Bauform besonders langlebig ist. Außerdem ist es eine Aufgabe der vorliegenden Anmeldung, ein entsprechend vorteilhaftes Verfahren zur Herstellung eines Pumpengehäuses vorzuschlagen.

Diese Aufgaben werden gelöst durch eine Blutpumpe mit den Merkmalen des unabhängigen Anspruchs 1 und durch ein Verfahren mit den Merkmalen eines weiteren Anspruchs. Vorteilhafte Weiterbildungen ergeben sich mit den Merkmalen der abhängigen Ansprüche und der Ausführungsbeispiele.

Die vorgeschlagene Blutpumpe ist geeignet zum Unterstützen einer Herzfunktion. Die Blutpumpe umfasst ein implantierbares Pumpengehäuse. Außerdem umfasst die Blutpumpe einen faseroptischen Sensor mit einer Lichtleitfaser. Die Lichtleitfaser ist zumindest teilweise in einem Gehäuseteil des Pumpengehäuses aufgenommen.

Dadurch, dass die Lichtleitfaser in dem Gehäuseteil des Pumpengehäuses der Blutpumpe aufgenommen ist, kann eine besonders robuste und kompakte Bauform der mit einem Sensor ausgestatteten Blutpumpe erreicht werden. In typischen Ausführungen weist der faseroptische Sensor auch eine Lichtquelle, einen Lichtdetektor und/oder eine Auswerteeinheit auf oder ist damit verbunden bzw. verbindbar. Die Lichtleitfaser kann hierbei einer Übertragung einer optischen Größe, die eine vom Sensor zu erfassende Messgröße repräsentiert, zwischen einem eigentlichen Messort und der Auswerteeinheit dienen. Die Lichtquelle ist in der Regel eingerichtet, Licht in die Lichtleitfaser einzukoppeln. Die Lichtquelle kann in einigen Ausführungen in dem Pumpengehäuse aufgenommen und/oder mit diesem starr verbunden sein. Der Lichtdetektor ist in der Regel eingerichtet, Licht zu detektieren, das aus der Lichtleitfaser heraustritt. Der Lichtdetektor kann in einigen Ausführungen in dem Pumpengehäuse aufgenommen und/oder mit diesem starr verbunden sein. Die Auswerteeinheit kann beispielsweise ebenfalls in dem Pumpengehäuse aufgenommen und/oder mit diesem starr verbunden sein. Die Auswerteeinheit ist typischerweise mit dem Lichtdetektor verbunden und kann zur Datenverarbeitung eingerichtet sein und hierfür beispielsweise einen Prozessor und einen Datenspeicher aufweisen. Der Messort liegt in der Regel im der Peripherie des Pumpengehäuses, beispielsweise auf einer Außenseite des Pumpengehäuses oder auf einer Innenseite des Pumpengehäuses. Indem die vorgeschlagene Blutpumpe einen faseroptischen Sensor mit einer Lichtleitfaser aufweist, kann der Messort des Sensors in besonders kompakter Weise festgelegt werden, ohne dass erhebliche Modifikationen der Blutpumpe zum Vorsehen des Sensors notwendig sind.

Ein Problem, das bei bekannten Sensortypen von Blutpumpen auftreten kann und das durch die vorgeschlagene Blutpumpe vermieden wird, ist eine unzureichende Abdichtung zwischen einem Messaufnehmer bzw. einem Übertragungskanal und einer mit dem Messaufnehmer über den Übertragungskanal verbundenen Auswerteeinheit. Beispielsweise kann bei den bekannten Sensortypen der Übertragungskanal einer elektrischen oder pneumatischen Übertragung einer Größe, die eine vom Sensor zu erfassende Messgröße repräsentiert, zwischen einem eigentlichen Messort und der Auswerteeinheit dienen. Wenn die Auswerteeinheit beispielsweise in einem Pumpengehäuse aufgenommen ist, kann die Abdichtung zwischen dem Übertragungskanal bzw. dem Messaufnehmer und einem den Messaufnehmer bzw. den Übertragungskanal aufnehmenden Gehäuseteil des Pumpengehäuses beispielsweise nur in unzureichender Weise durch eine Kunststoffdichtung erfolgen, da mit der Kunststoffdichtung in Kontakt tretendes Blut bei implantierter Blutpumpe für gängige Typen von Kunststoffdichtungen ein aggressives Medium darstellen kann, das einerseits das Material der Kunststoffdichtung angreifen kann und andererseits durch dieses Material hindurchdiffundieren kann, was zu einer Beeinträchtigung der Langzeitstabilität bekannter Blutpumpen führt.

Durch das Vorsehen einer Lichtleitfaser, die in dem Gehäuseteil des Pumpengehäuses aufgenommen ist, kann auf den Einsatz herkömmlicher Kunststoffdichtungen verzichtet werden, so dass die vorgeschlagene Blutpumpe besonders langzeitstabil ist.

Die Anmeldung betrifft auch ein entsprechend vorteilhaftes Verfahren zur Herstellung eines Pumpengehäuses einer Blutpumpe. Bei dem vorgeschlagenen Verfahren wird eine Lichtleitfaser bereitgestellt. Außerdem wird ein erstes Metallstück bereitgestellt. Die Lichtleitfaser wird auf dem ersten Metallstück angeordnet. Anschließend wird die Lichtleitfaser mit einem zweiten Metallstück zumindest teilweise überdeckt. Weiterhin kann die Lichtleitfaser in die Metallstücke eingebettet werden. Hierzu kann Ultraschallenergie in das erste und/oder das zweite Metallstück eingebracht werden, wobei aber auch beispielsweise ein Einbringen von Wärmeenergie möglich ist. Dadurch können das erste und das zweite Metallstück verbunden werden. Die Metallstücke können aus demselben Material bestehen.

Des Weiteren kann die Lichtleitfaser derart in die Metallstücke eingeschlossen werden, dass die Lichtleitfaser zumindest teilweise in ein aus dem ersten Metallstück und dem zweiten Metallstück gebildetes Gehäuseteil umlaufend, insbesondere lückenfrei, eingebettet wird. In einigen Ausführungen ist bzw. wird die Lichtleitfaser mit den Metallstücken bzw. dem Gehäuseteil stoffschlüssig verbunden. Hierbei können Bereiche der Metallstücke einen die Lichtleitfaser umlaufenden Ring bilden. Ein Bereich des Gehäuseteils kann zum Beispiel einen die Lichtleitfaser umlaufenden und geschlossenen Ring bilden. Auf diese Weise erzeugen das Gehäuseteil und die Lichtleitfaser eine besonders zuverlässige Abdichtung. Vorteilhafterweise ist der die Lichtleitfaser umgebende Bereich des Gehäuseteils einstückig ausgebildet, sodass eine besonders zuverlässige Abdichtung erreicht wird.

Die Lichtleitfaser weist typischerweise einen lichtführenden Kern auf. Der lichtführende Kern kann in einigen Ausführungen Kunststofffasern, beispielsweise Acrylharz-Fasern, umfassen. Besonders in Bezug auf die oben beschriebenen Dichtungseigenschaften und die Langzeitstabilität der Blutpumpe ist es jedoch vorteilhaft, wenn der lichtführende Kern Glasfasern enthält, so dass eine Diffusion von Blut in den lichtführenden Kern vermieden oder reduziert wird.

In einigen Ausführungen ist der lichtführende Kern der Lichtleitfaser ummantelt. Beispielsweise kann der lichtführende Kern beschichtet sein. Geeignet ist typischerweise eine Ummantelung aus einem Metall, um die oben geschilderten Probleme kunststoffbasierter Materialien zu umgehen. Zudem kann auf diese Weise eine besonders gute Abdichtung zwischen der Lichtleitfaser und dem Gehäuseteil erreicht werden. Beispielsweise kann die Ummantelung dasselbe Material wie der Gehäuseteil des Pumpengehäuses umfassen oder aus diesem bestehen. In der Regel sind Materialien zumindest derjenigen Teile des Gehäuseteils bzw. der Ummantelung, die beim Einsatz der Blutpumpe im Körper eines Patienten mit Blut in Kontakt kommen, biokompatibel. Die Ummantelung der Lichtleitfaser und/oder das Gehäuseteil, insbesondere die Metallstücke, können vorteilhafterweise Titan, insbesondere eine Titanlegierung, enthalten. Beispielsweise können das Gehäuseteil, insbesondere die Metallstücke, bzw. die Lichtleitfaser aus Titan oder aus der Titanlegierung gefertigt sein. Auf diese Weise sind das Gehäuseteil bzw. die Ummantelung biokompatibel und besonders korrosionsbeständig.

In der Regel ist eine Stirnseite eines in das Gehäuseteil des Pumpengehäuses aufgenommenen Faserendes der Lichtleitfaser nicht vollständig von dem Gehäuseteil bedeckt. Ein mit dem Faserende verbundener oder durch das Faserende gebildeter Messaufnehmer des faseroptischen Sensors ist hierbei in der Regel nicht vollständig von dem Gehäuseteil bedeckt, so dass dieser eingerichtet ist, mit einem Körperinneren des Patienten, insbesondere mit Blut des Patienten, in Kontakt zu treten. Die vorgeschlagene Blutpumpe kann dadurch besonders korrosionsbeständig sein, dass das Faserende derart in das Gehäuseteil eingebettet ist, dass das Faserende und das Gehäuseteil eine kunststofffreie Abdichtung ausbilden. Hierbei wird vermieden, dass Blut in Teile der Abdichtung eindringt, was beispielsweise bei Abdichtungen mit Polymerteilen auftreten kann.

Die Blutpumpe weist in der Regel einen Motor auf. Der Motor umfasst typischerweise einen Stator, beispielsweise Wicklungen umfassend, und einen Rotor, beispielsweise einen Permanentmagneten umfassend. Der Motor ist typischerweise zumindest teilweise, insbesondere vollständig, in dem Pumpengehäuse aufgenommen. Zudem umschließt und/oder definiert das Pumpengehäuse einen Strömungskanal zum Fördern von Blut. Der Rotor, der als Förderelement ausgebildet oder mit einem Förderelement verbunden sein kann, ist typischerweise in dem Strömungskanal angeordnet. Das Förderelement ist in der Regel ebenfalls im Strömungskanal angeordnet. Das Pumpengehäuse weist in der Regel einen von dem Förderelement stromaufwärts angeordneten Einlass und einen von dem Förderelement stromabwärts angeordneten Auslass auf. Der Einlass kann eine Einlasskanüle umfassen. Das Pumpengehäuse kann zudem eine Kammerhälfte aufweisen. Ein Bereich der Kammerhälfte kann den Auslass des Pumpengehäuses bilden. Ein durch die Kammerhälfte begrenzter Teil des Strömungskanals kann im Wesentlichen spiralförmig sein.

In der Regel wird der von dem faseroptischen Sensor gemessene Messwert im Bereich einer Stirnseite der Lichtleitfaser erfasst. Es kann in einigen Ausführungen vorgesehen sein, dass die Lichtleitfaser mit ihrer Stirnseite dem Strömungskanal zugewandt ist und/oder in den Strömungskanal zum Fördern von Blut mündet. Insbesondere kann es vorgesehen sein, dass die Lichtleitfaser so angeordnet ist, dass der Messort im Strömungskanal liegt. Es kann in einigen Ausführungen weiterhin vorgesehen sein, dass die Stirnseite der Lichtleitfaser durch den Messaufnehmer des Sensors von dem Strömungskanal getrennt ist. Es kann aber auch vorgesehen sein, dass die Stirnseite der Lichtleitfaser eingerichtet ist, direkt mit dem Strömungskanal in Kontakt steht. Beispielsweise kann der lichtführende Kern der Lichtleitfaser eingerichtet und angeordnet sein, mit Blut des Patienten in Kontakt zu treten. Der Strömungskanal kann hierbei zumindest teilweise von einer innenliegenden Seite des Gehäuseteils begrenzt sein. Wenn die Lichtleitfaser mit Ihrer Stirnseite dem Strömungskanal zugewandt ist, kann die Blutpumpe besonders vorteilhaft sein, da der Sensor aufgrund einer vergleichsweise kleinen Abmessung der Lichtleitfaser im Vergleich zu anderen Sensorarten eine nur auf eine geringe Fläche beschränkte Modifikation der Begrenzung des Strömungskanals benötigt. Die Modifikation der Begrenzung des Strömungskanals kann im Allgemeinen durch einen durch den Sensor bedingten Unterschied der Form oder des Materials einer Wandung des Strömungskanals bedingt sein. Somit kann die Blutpumpe durch Verwendung der Lichtleitfaser, die insbesondere im Vergleich zu herkömmlichen lichtdurchlässigen Fenstern einen geringen Querschnitt haben kann, besonders blutschonend gestaltet werden. Ein Querschnitt der Lichtleitfaser kann beispielsweise höchstens 1 mm, vorzugsweise höchstens 500 µm und besonders vorzugsweise höchstens 200 µm, betragen.

Es kann in einigen Ausführungen vorgesehen sein, dass die Stirnseite der Lichtleitfaser an einem in Bezug zu dem Förderelement stromaufwärtsliegenden Abschnitt des Strömungskanals angeordnet ist. Beispielsweise kann die Lichtleitfaser an dem stromaufwärtsliegenden Abschnitt in den Strömungskanal münden oder es kann der Messaufnehmer in diesem Abschnitt angeordnet sein. In diesem Fall ist eine Messung von Blutparametern unter Verwendung des faseroptischen Sensors besonders zuverlässig, da das Blut am Einlass noch nicht in Kontakt mit dem Förderelement getreten ist und durch diesen noch nicht beeinflusst oder geschädigt ist. Das Gehäuseteil, in dem die Lichtleitfaser aufgenommen ist, kann beispielsweise durch die Einlasskanüle des Pumpengehäuses gebildet werden. In anderen Ausführungen mündet die Lichtleitfaser an einem stromabwärtsliegenden Abschnitt in den Strömungskanal oder der Messaufnehmer ist in diesem Abschnitt angeordnet. Hierbei kann der Gehäuseteil, in dem die Lichtleitfaser aufgenommen ist, beispielsweise durch die Kammerhälfte des Pumpengehäuses gebildet werden.

Die Blutpumpe kann in einigen Ausführungen eine Regeleinrichtung umfassen. Die Regeleinrichtung kann mit der Auswerteeinheit verbunden sein. Die Regeleinrichtung ist typischerweise zur Datenverarbeitung eingerichtet und weist hierfür beispielsweise einen Prozessor und einen Datenspeicher auf. Die Regeleinrichtung kann mit dem faseroptischen Sensor und dem Motor der Blutpumpe verbunden sein. Die Regeleinrichtung kann zudem eingerichtet sein, eine Förderleistung der Blutpumpe anhand von Messwerten, die von dem faseroptischen Sensor ermittelt wurden, einzustellen. Bei einem Verfahren zur Herzunterstützung kann beispielsweise zunächst eine wie oben oder unten beschriebene Blutpumpe bereitgestellt und implantiert werden. Außerdem kann eine Förderleistung der Blutpumpe anhand der Messwerte, die von dem faseroptischen Sensor ermittelt wurden, eingestellt werden.

Es ist in einigen Ausführungen vorgesehen, dass der faseroptische Sensor ein Sensor zur Bestimmung von Blutparametern ist. Beispielsweise kann der Sensor ein Blutdrucksensor sein. Beispielsweise kann der Blutdrucksensor eine Fabry-Perot-Kavität oder ein Faser-Bragg-Gitter aufweisen. Insbesondere wenn der faseroptische Sensor ein Blutdrucksensor ist, kann die Auswerteeinheit zur Bestimmung eines Flusses des geförderten Blutes anhand der vom faseroptischen Sensor gemessenen Blutdruckwerte eingerichtet sein. Hierbei kann die Regeleinrichtung eingerichtet sein, den Motor der Blutpumpe anhand der bestimmten Werte des Flusses anzusteuern, beispielsweise indem die Regeleinrichtung den Fluss einem gewünschten Soll-Fluss anpasst.

Der faseroptische Sensor kann in einigen Ausführungen eingerichtet sein, neben dem Blutdruck oder zusätzlich zu dem Blutdruck weitere Blutparameter zu bestimmen, wie beispielsweise einen Hämatokritwert, einen Blutzuckergehalt und/oder einen Blutsauerstoffgehalt. Außerdem kann es vorgesehen sein, dass der faseroptische Sensor ein Sensor zur Thrombendetektion ist.

In einigen Ausführungen ist der faseroptische Sensor eingerichtet, eine Position des Förderelementes zu detektieren. Zu diesem Zweck kann das Faserende der Lichtleitfaser im Bereich des Förderelementes angeordnet sein. Die Stirnseite der Lichtleitfaser kann beispielsweise auf das Förderelement ausgerichtet sein. Die Auswerteeinheit kann beispielsweise eingerichtet sein, die Position des Förderelementes anhand von von dem Förderelement zurückgestreutem und detektiertem Licht zu bestimmen. Auf diese Weise kann beispielsweise eine Taumelbewegung des Förderelements beim Betrieb der Blutpumpe detektiert werden, anhand der die Auswerteeinheit eine Viskosität des Blutes bestimmen kann. Die Regeleinrichtung kann wiederum eingerichtet sein, den Motor der Blutpumpe anhand der bestimmten Viskosität des Blutes anzusteuern.

Es kann vorgesehen sein, dass die Lichtleitfaser mit ihrer Stirnseite einer von einer außenliegenden Seite des Gehäuseteils begrenzten Außenseite des Pumpengehäuses zugewandt ist. Beispielsweise kann vorgesehen sein, dass die Lichtleitfaser mit ihrer Stirnseite in eine von einer außenliegenden Seite des Gehäuseteils begrenzte Außenseite des Pumpengehäuses mündet. Insbesondere kann es vorgesehen sein, dass die Lichtleitfaser so angeordnet ist, dass der Messort auf der Außenseite des Pumpengehäuses liegt.

Wenn die Stirnseite der Lichtleitfaser einem inneren Gehäuseteil, beispielsweise dem Strömungskanal, oder einem äußeren Gehäuseteil zugewandt sein soll, kann bei dem Herstellungsverfahren für das Pumpengehäuse ein subtraktiver Fertigungsschritt zum Ausbilden der Stirnseite der Lichtleitfaser vorgesehen sein. Beispielsweise kann nach dem Einbetten der Lichtleitfaser in die Metallstücke Material von den Metallstücken und/oder der Lichtleitfaser entfernt, insbesondere geschliffen, werden, so dass die Stirnseite der Lichtleitfaser mit den Metallstücken bündig abschließt. Auf diese Weise kann erreicht werden, dass die Lichtleitfaser mit dem Gehäuseteil eine gemeinsame, glatte Oberfläche ausbildet, so dass an dieser beim Einsatz der Blutpumpe vorbeiströmendes Blut nicht oder nur in geringem Maße geschädigt wird. Der faseroptische Sensor kann somit eine gemeinsame geschliffene Oberfläche aufweisen, die bereichsweise durch eine geschliffene Stirnseite der Lichtleifaser und eine mit der Stirnseite fluchtende und diese umlaufende geschliffene Oberfläche des Pumpengehäuses aufweist. Die gemeinsame geschliffene Oberfläche kann beispielsweise einen Teil der Begrenzung des Strömungskanals bilden.

Es kann in einigen Ausführungen außerdem vorgesehen sein, dass der faseroptische Sensor eine zweite Lichtleitfaser aufweist. Die Lichtleitfasern können derart angeordnet und ausgebildet sein, dass der faseroptische Sensor ein faseroptischer Transmissionssensor ist. Hierbei können beide Lichtleitfasern beispielsweise in den Strömungskanal münden. Typischerweise sind Stirnseiten der Lichtleitfasern hierbei einander derart zugewandt, dass Licht, das in die erste Lichtleitfaser eingekoppelt wird, nach dem Durchtritt durch den Strömungskanal in die Stirnseite der zweiten Lichtleitfaser eingekoppelt wird. Nach dem Durchtritt durch die zweite Lichtleitfaser kann das Licht mit einem Lichtdetektor detektiert und durch die Auswerteeinheit ausgewertet werden. Hierdurch sind Rückschlüsse auf Blutparameter wie beispielsweise einen Hämatokritwert oder eine Thrombendichte bzw. ein Vorhandensein von Thromben möglich.

Die Blutpumpe kann in einigen Ausführungen zumindest zwei faseroptische Sensoren aufweisen, die wie oben oder unten beschrieben ausgebildet sein können. Beispielsweise kann die Blutpumpe einen zweiten faseroptischen Sensor aufweisen. Es kann vorgesehen sein, dass der erste faseroptische Sensor zur Messung eines Blutdruckes in dem von der innenliegenden Seite des Gehäuseteils begrenzten Strömungskanal zum Fördern von Blut eingerichtet ist. Der zweite faseroptische Sensor kann zur Messung eines Blutdruckes entweder in dem Strömungskanal oder an einer Außenseite des Pumpengehäuses eingerichtet sein.

Sofern der zweite faseroptische Sensor zur Messung des Blutdruckes in dem Strömungskanal eingerichtet ist, ist es besonders vorteilhaft, wenn einer der Sensoren in Bezug auf das Förderelement stromaufwärts und der zweite der Sensoren in Bezug auf das Förderelement stromabwärts angeordnet ist. Auf diese Weise kann die Auswerteeinheit in besonders einfacher Weise aus den beiden gemessenen Blutdruckwerten den Fluss des geförderten Blutes bestimmen.

Wenn der zweite faseroptische Sensor oder ein weiterer faseroptischer Sensor, der wie der erste faseroptische Sensor und/oder der zweite faseroptische Sensor ausgebildet sein kann, zur Bestimmung des Blutdruckes an einer Außenseite des Pumpengehäuses eingerichtet ist, kann der Blutdruck auf besonders genaue Weise bestimmt werden, indem die Auswerteeinheit eingerichtet ist, Störeinflüsse auf die Blutdruckmessung anhand der an der Außenseite des Pumpengehäuses gemessenen Blutdruckwerte zu bestimmen und die in dem Strömungskanal bestimmten Blutdruckwerte anhand der Störeinflüsse zu korrigieren.

Oben oder unten im Hinblick auf die Blutpumpe zum Unterstützen der Herzfunktion beschriebene Merkmale sind auf das Verfahren zur Herstellung des Pumpengehäuses anwendbar umgekehrt.

Ausführungsbeispiele werden nachfolgend anhand der Abbildungen beschrieben. Es zeigen
- Fig. 1: eine schematische Ansicht einer Blutpumpe, die im Körper eines Patienten implantiert ist,
- Fig. 2: eine schematische Ansicht eines faseroptischen Sensors der Blutpumpe,
- Fign. 3(a) bis (g): schematische Darstellungen von Verfahrensschritten bei der Herstellung der Blutpumpe,
- Fign. 4(a) bis (d): schematische Ansichten eines Pumpengehäuses der Blutpumpe,
- Fig. 5: eine schematische Ansicht eines weiteren faseroptischen Sensors der Blutpumpe,
- Fig. 6(a) und (b): schematische Ansichten von als Blutdrucksensoren ausgeführten faseroptischen Sensoren der Blutpumpe,
- Fig. 7: eine schematische Ansicht eines als Sensor zur Bestimmung einer Rotorposition ausgeführten faseroptischen Sensors der Blutpumpe,
- Fign. 8(a) und (b): schematische Ansichten von als Sensoren zur Thrombendetektion, eines Hämatokritwerts, eines Blutzuckerspiegels bzw. eines Blutsauerstoffwerts ausgeführten faseroptischen Sensoren der Blutpumpe.

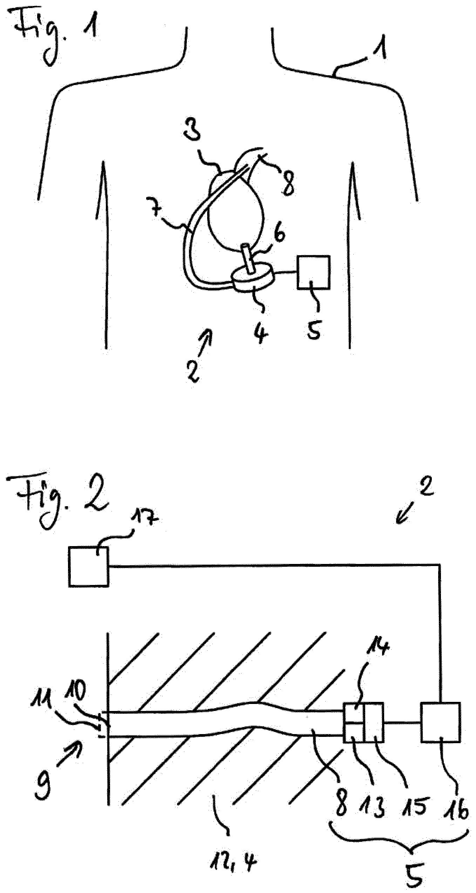

Figur 1 zeigt schematisch einen Körper 1 eines Patienten, in dem eine Blutpumpe 2 zur Unterstützung einer Funktion des Herzens 3 implantiert ist. Die Blutpumpe 2 weist einen typischerweise als Elektromotor mit einem rotierbaren Förderelement ausgeführten Motor auf, der in einem Pumpengehäuse 4 der Blutpumpe 2 aufgenommen ist. Das Pumpengehäuse 4 ist mit einem Steuergerät 5 verbunden, das ebenfalls implantiert sein kann, wie schematisch gezeigt ist. Das Steuergerät 5 kann in einigen Ausführungen vollständig oder teilweise ebenfalls in dem implantierten Pumpengehäuse 4 aufgenommen sein. In anderen Ausführungen ist das Steuergerät 5 extrakorporal angeordnet. Das Pumpengehäuse 4 umfasst außerdem einen mit einer Einlasskanüle des Pumpengehäuses 4 verbundenen Einlasskanal 6, über den Blut aus einer Kammer des Herzens 3 entnommen und über eine Kanüle 7 in ein Blutgefäß 8 gefördert werden kann. Das Steuergerät 5 ist eingerichtet, den Motor der Blutpumpe 2 zum Fördern des Blutes anzusteuern.

Figur 2 zeigt eine schematische Darstellung der Blutpumpe 2. Wiederkehrende Merkmale sind in dieser Abbildung und in den nachfolgenden Abbildungen mit den gleichen Bezugszeichen versehen. Die Blutpumpe 2 weist einen faseroptischen Sensor mit einer Lichtleitfaser 8 auf. Der faseroptische Sensor ist eingerichtet zur Messung von Messwerten wie beispielsweise einem Blutdruck, einem Hämatokritwert, einer Thrombendichte, einem Blutzuckerspiegel, einem Blutsauerstoffwert oder einer Rotorposition an einem Messort 9. Der Messort 9 wird bestimmt durch die Position einer Stirnseite 10 der Lichtleitfaser 8 oder einen gegebenenfalls an dieser Stirnseite 10 angeordneten Messaufnehmer 11. Die Lichtleitfaser 8 oder zumindest ein Teil der Lichtleitfaser 8 ist in einem Gehäuseteil 12, beispielsweise einem Titanteil, des Pumpengehäuses 4 eingebettet, so dass das Gehäuseteil 12 die Lichtleitfaser 8 zumindest in einem Bereich vollständig umschließt und so dass die Lichtleitfaser 8 und das Gehäuseteil 12 blutdicht und dabei kunststofffrei sind. Ein von der Stirnseite 10 abgewandtes Ende der Lichtleitfaser 8 mündet in eine Lichtquelle 13, beispielsweise eine Leuchtdiode, und einen Lichtdetektor 14. Die Lichtquelle 13 ist eingerichtet, Licht abzustrahlen und in die Lichtleitfaser 8 einzukoppeln, während der Lichtdetektor 14 eingerichtet ist, aus der Lichtleitfaser 8 austretendes Licht zu detektieren. Die Lichtquelle 13 und der Lichtdetektor 14 sind mit einer elektronischen Auswerteeinheit 15 verbunden, die eingerichtet ist, die Lichtquelle 13 anzusteuern und den Lichtdetektor 14 auszulesen. Die Auswerteeinheit 15 ist eingerichtet, anhand des von dem Lichtdetektor 14 detektierten Lichtes die oben genannten Messwerte zu bestimmen. Außerdem ist die Auswerteeinheit 15 mit einer elektronischen Regeleinrichtung 16 verbunden. Anhand der Messwerte, die von der Auswerteeinheit 15 an die Regeleinrichtung 16 übertragen wurden, kann die Regeleinrichtung 16 den mit dieser verbundenen Motor 17 der Blutpumpe 2 ansteuern, beispielsweise indem die Regeleinrichtung 16 einen Stromfluss in Windungen des Stators anpasst.

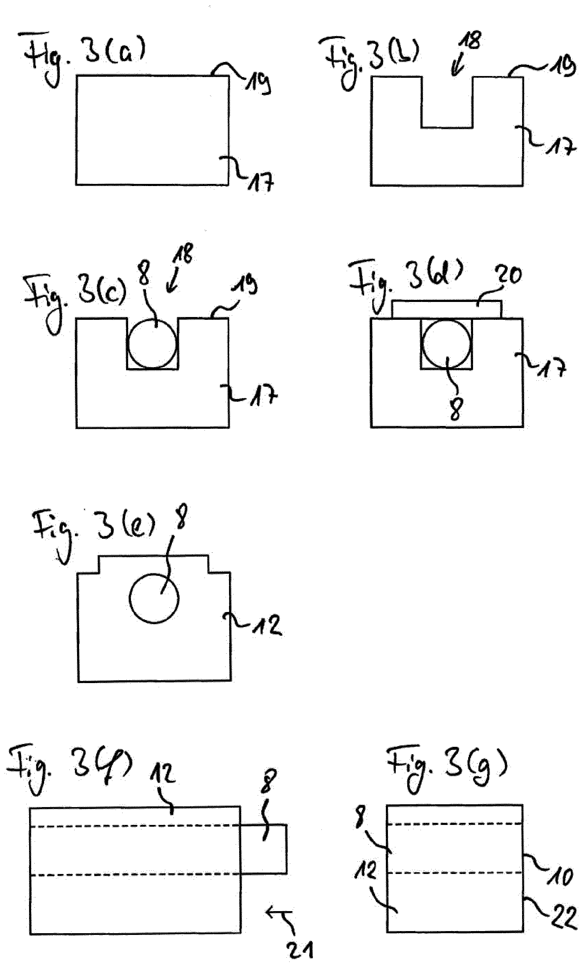

Figuren 3(a) bis (g) illustrieren schematisch unterschiedliche Verfahrensschritte während einer Herstellung der Blutpumpe 2 und des Pumpengehäuses 4.

Hierbei wird zunächst ein erstes Metallstück 17, beispielsweise ein biokompatibles Titan- oder Titanlegierungsstück, bereitgestellt, das nach der Herstellung ein Stück des Gehäuseteils 12 des Pumpengehäuses 4, beispielsweise eine Kammerhälfte oder eine Einlasskanüle, bilden soll. Anschließend wird eine Vertiefung 18, beispielsweise eine Rille, auf einer Oberfläche 19 des Metallstücks 17 erzeugt. Die Vertiefung 18 kann beispielsweise gefräst werden. In einem weiteren Schritt wird die Lichtleitfaser 8 in die Vertiefung 18 eingelegt und durch ein zweites Metallstück 20, das aus demselben Material besteht wie das erste Metallstück 17, abgedeckt, wie Fig. 3(d) zeigt.

Anschließend wird die Lichtleitfaser 8 in die Metallstücke 17, 20 eingebettet so dass die Metallstücke 17, 20 die Lichtleitfaser 8 lückenlos umschließen, wie Fig. 3(e) zeigt. Insbesondere ist die Lichtleitfaser nach der Herstellung zumindest teilweise in das Gehäuseteil umlaufend eingebettet. Hierzu kann eine Ultraschallsonotrode verwendet werden, die Ultraschallenergie in das erste und das zweite Metallstück 17, 20 einbringt. Durch das Einbringen der Ultraschallenergie werden zudem die Metallstücke 17, 20 miteinander verbunden, so dass diese ein einstückiges Gehäuseteil 12 ausbilden. Die Lichtleitfaser 8 kann in einigen Ausführungen einen lichtführenden Kern aus Glasfasern bzw. Glasfaserbündeln und zusätzlich eine Metallbeschichtung, insbesondere aus Titan oder einer Titanlegierung, aufweisen, so dass beim Einbetten der Lichtleitfaser 8 eine stoffschlüssige Verbindung zwischen der Beschichtung der Lichtleitfaser 8 und den Metallstücken 17, 10 erzeugt wird.

Figuren 3(f) und (g) zeigen Seitenansichten von einer Anordnung nach dem Einbetten. Hierbei ist ein Teil der Lichtleitfaser 8 vollständig in das aus den Metallstücken 17, 20 gebildete Gehäuseteil 12 eingebettet, dieser Teil ist in der Abbildung lediglich mithilfe von gestrichelten Linien gezeigt. Figur 3(f) entspricht hierbei der in Fig. 3(e) gezeigten Anordnung. In einem weiteren Schritt wird die Anordnung von einer Seite in Richtung des Pfeils mit dem Bezugszeichen 21 geschliffen. Nach dem Schleifen bilden die Lichtleitfaser 8 und der Gehäuseteil 17 eine gemeinsame geschliffene Oberfläche 22 aus, die eine Außenwand des fertig hergestellten Pumpengehäuses 4 oder eine Begrenzung eines blutführenden Strömungskanals im Inneren des Pumpengehäuses 4 bilden kann.

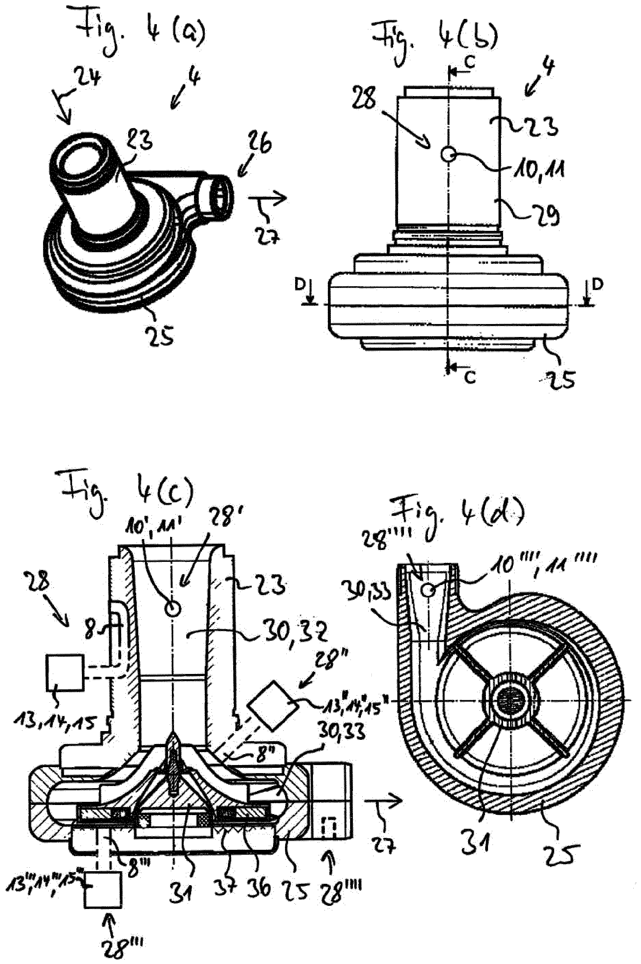

Figuren 4(a) bis (d) zeigen unterschiedliche Ansichten des Pumpengehäuses 4 der Blutpumpe 2. In Fig. 4(a) ist eine perspektivische Außenansicht des Pumpengehäuses 4 gezeigt. Das Pumpengehäuse 4 weist die Einlasskanüle 23 auf, in die beim Betrieb der Blutpumpe 2 Blut vom Herzen 3 in Richtung des Pfeils mit dem Bezugszeichen 24 einströmt. Außerdem weist das Pumpengehäuse 4 die Kammerhälfte 25 auf, durch die eine untere Teilbegrenzung einer Auslassöffnung 26 bildet, aus der beim Betrieb der Blutpumpe 2 Blut in Richtung des Pfeils mit dem Bezugszeichen 27 in die Kanüle 7 und das Blutgefäß 8 ausströmt.

Figur 4(b) zeigt eine Seitenansicht des Pumpengehäuses 4. In dieser Darstellung ist auch ein faseroptischer Sensor 28 gezeigt, der wie oben beschrieben ausgebildet ist und dessen Messort an einer Außenseite 29 der Einlasskanüle 23 des Pumpengehäuses 4 angeordnet ist. Die Stirnseite 10 der Lichtleitfaser 8 oder der ggfs. vorgesehene, die Stirnseite 10 überdeckende Messaufnehmer 11 des faseroptischen Sensors 28 ist hierbei bündig mit der Außenseite 29 der Einlasskanüle 23 ausgeführt.

Figuren 4(c) und (d) zeigen jeweils eine Querschnittsansicht durch das Pumpengehäuse 4 entlang einer in Fig. 4(b) mit dem Buchstaben C bzw. D gekennzeichneten Schnittlinie. Durch Innenwände des Pumpengehäuses 4 wird ein Strömungskanal 30 ausgebildet, in dem ein Rotor 31 aufgenommen ist. In dem dargestellten Beispiel ist ein Rotor 31 vorgesehen, der als Radialrotor ausgebildet ist. Der Rotor 31 ist in diesem Fall eingerichtet, in der Richtung 24 über die Einlasskanüle 23 axial einströmendes Blut in radialer Richtung zu beschleunigen und in der Richtung 27 in die Kanüle 7 zu leiten. Die hier beschriebene Blutpumpe 2 kann in anderen Ausführungen jedoch auch entsprechend als Axialpumpe mit einem axial fördernden Rotor ausgebildet sein. Der Rotor 31 unterteilt den Strömungskanal 30 in einen stromaufwärtsliegenden Abschnitt 32 und einen stromabwärtsliegenden Abschnitt 33. Der Rotor 31 umfasst einen Rotormagneten 36, der eingerichtet ist, zum Antrieb des Motors 17 mit in der Kammerhälfte 25 aufgenommenen Statorwindungen 37 zusammenzuwirken.

Die Lichtleitfaser 8 des faseroptischen Sensors 28 ist gemäß dem oben beschriebenen Verfahren in die Einlasskanüle 23 des Pumpengehäuses 4 eingebettet. Zudem sind schematisch die Lichtquelle 13, der Lichtdetektor 14 und die Auswerteeinheit 15 dargestellt, die mit der Lichtleitfaser 8 verbunden sind und beispielsweise außerhalb des Pumpengehäuses 4, insbesondere außerhalb des Körpers 1 des Patienten, angeordnet oder in einigen Ausführungen in das Pumpengehäuse 4 integriert sein können.

Neben dem beschriebenen faseroptischen Sensor 28 weist die Blutpumpe 2 weitere faseroptische Sensoren 28', 28", 28''', 28"" auf, die entsprechend dem beschriebenen faseroptischen Sensor 28 ausgebildet sein können. Eine Stirnseite 10' der Lichtleitfaser oder ein die Stirnseite 10' überdeckender Messaufnehmer 11' der Sensors mit dem Bezugszeichen 28' ist so angeordnet und ausgerichtet, dass der Messort dieses Sensors 28' im stromaufwärtsliegenden Abschnitt 32 des Strömungskanals 30 liegt.

Die Sensoren mit den Bezugszeichen 28" und 28'" sind auf den Rotor 31 ausgerichtet und können eingerichtet sein, eine Position des Rotors 31 zu bestimmen. Diese Sensoren 28", 28'" können aber wie alle anderen oben oder unten beschriebenen Sensoren auch als Messsensoren für einen Blutdruck, einen Hämatokritwert, eine Thrombendichte, einen Blutzuckerspiegel oder einen Blutsauerstoffwert ausgebildet sein. Die Lichtleitfaser 8" eines der Sensoren 28" ist hierbei in die Einlasskanüle 23 eingebettet während die Lichtleitfaser 8'" des anderen Sensors 28'" in die Kammerhälfte 25 eingebettet ist.

In Fig. 4(c) ist die Position eines weiteren faseroptischen Sensors 28"", der wie die anderen oben beschriebenen Sensoren ausgebildet sein kann, durch eine gestrichelte Linie gekennzeichnet, wobei ein genauer Messort anhand der Stirnseite 10"" bzw. eines die Stirnseite 10"" überdeckenden und gegebenenfalls vorhandenen Messaufnehmers 11"" im stromabwärtsliegenden Abschnitt 33 des Strömungskanals 30 in der Fig. 4(d) zu erkennen ist.

Die Auswerteeinheiten der Sensoren 28, 28', 28", 28'", 28"" können beispielsweise separate Bauteile sein, wie in Fig. 4(c) anhand der beispielhaft gezeigten Auswerteeinheiten 15, 15" und 15'" dargestellt ist. Es können aber auch einige oder alle der Auswerteeinheiten in einer einzelnen elektronischen Einheit zusammengefasst sein. Die Auswerteeinheit bzw. die Auswerteeinheiten können wir oben beschrieben mit der Regeleinrichtung 16 verbunden sein. Insbesondere wenn die Sensoren mit den Bezugszeichen 28, 28' und 28"" als Blutdrucksensoren ausgeführt sind, kann die Regeleinrichtung 16 eingerichtet sein, den Motor 17 der Blutpumpe 2 anhand von Differenzen beispielsweise der von den Sensoren mit den Bezugszeichen 28' und 28"" erfassten Blutdruckwerte und/oder Differenzen der von den Sensoren mit den Bezugszeichen 28 und 28' oder 28 und 28"" erfassten Blutdruckwerte anzusteuern.

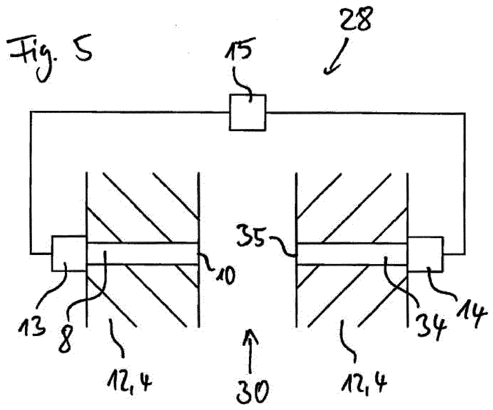

Figur 5 zeigt eine weitere Ausgestaltung eines faseroptischen Sensors 28, der als Transmissionssensor ausgebildet ist. Dieser Sensor weist neben der Lichtleitfaser 8 eine weitere Lichtleitfaser 34 auf. Die Lichtleitfasern 8, 34 sind in dem dargestellten Beispiel jeweils in den Gehäuseteil 12 des Pumpengehäuses 4 wie oben beschrieben eingebettet, wobei der Gehäuseteil 12 einen blutführenden Bereich, beispielsweise den Strömungskanal 30, teilweise umschließt.

Die Lichtquelle 13 ist so angeordnet, dass durch diese ausgestrahltes Licht in die Lichtleitfaser 8 eingekoppelt werden kann. Die Lichtleitfasern 8, 34 sind mit den Stirnseiten 10, 35 so angeordnet und ausgerichtet, dass Licht, das aus der Stirnseite 10 der einen Lichtleitfaser 8 austritt, durch den Strömungskanal 30 hindurchtritt und über die Stirnseite 35 der zweiten Lichtleitfaser 34 in diese hineintritt. Über den mit einem anderen Ende der zweiten Lichtleitfaser 34 verbundenen Lichtdetektor 14 kann das Licht anschließend detektiert werden. Die Auswerteeinheit 15 ist wie oben beschrieben mit dem Lichtdetektor 14 und mit der Lichtquelle 13 verbunden.

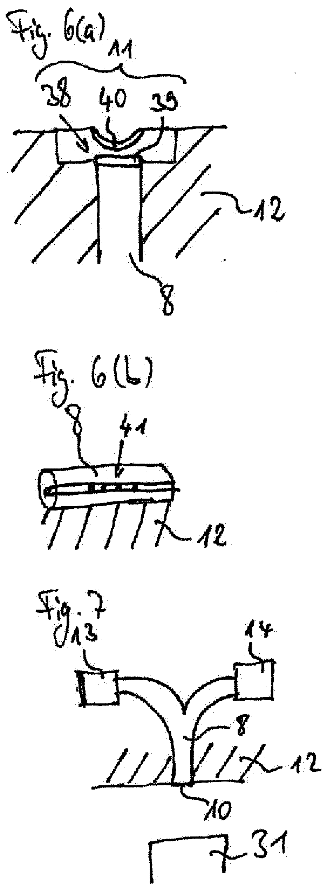

In Fig. 6(a) ist schematisch ein eine Fabry-Perot-Kavität 38 aufweisender faseroptischer Sensor der Blutpumpe 2 zur Blutdruckmessung gezeigt. Bei der Blutdruckmessung unter Verwendung dieses Sensors umfasst die Messzelle bzw. der Messaufnehmer 11 zwei teilreflektierende Spiegel 39, 40, die sich durch eine hohe Reflektivität auszeichnen können und die miteinander einen optischen Resonator bilden. Ein an den Messort 9 des Sensors angrenzender Spiegel 40 ist biegsam, beispielsweise als Membran, ausgeführt. Das Spektrum der Anordnung zeigt schmale Intensitätsmaxima für Wellenlängen, welche die Resonanzbedingung erfüllen, während andere Spektralbereiche nahezu vollständig ausgelöscht werden. Aus dem durch die Lichtleitfaser 8 auf den Messaufnehmer 11 treffenden und von diesem reflektierten Lichtspektrum kann durch die Auswerteeinheit 15 auf den Blutdruck geschlossen werden.

Figur 6 (b) zeigt schematisch einen faseroptischen Sensor der Blutpumpe 2, der zur Blutdruckmessung ein Faser-Bragg-Gitter 41 aufweist. Das Faser-Bragg-Gitter 41 umfasst in den Lichtwellenleiter 8, der bei seiner Verwendung in diesem Beispiel an Blut des Patienten angrenzt, eingeschriebene optische Interferenzfilter. Wellenlängen, die innerhalb einer Filterbandbreite liegen, werden durch das Faser-Bragg-Gitter 41 reflektiert. Durch eine blutdruckdruckinduzierte Längenänderung der Lichtleitfaser 8 ändert sich das reflektierte Spektrum, so dass die Auswerteeinheit 15 aus diesem Spektrum auf den Blutdruck schließen kann.

In Fig. 7 ist ein faseroptischer Sensor der Blutpumpe 2 zur Bestimmung einer Position des Rotors 31 gezeigt. Hierbei kann eine faseroptische Abstandsmessung Anwendung finden, die insbesondere bei lichtreflektierenden Oberflächen zuverlässige Ergebnisse liefert. Beispielsweise verläuft die Lichtleitfaser zu diesem Zweck zwischen der Lichtquelle 13 bzw. dem Lichtdetektor 14 und dem Strömungskanal 30 derart, dass die Stirnseite 10 dem Rotor 31 zugewandt ist. Aus einer Intensität des von dem Rotor 31 reflektierten Lichts kann durch die Auswerteeinheit 15 auf den Abstand bzw. die Position des Rotors 31 geschlossen werden. In einigen Ausführungen können die Lichtquelle 13 und der Lichtdetektor 14 auch jeweils mit einer Lichtleitfaser ausgestattet sein, wobei jeweilige Stirnseiten dieser Lichtleitfasern dem Rotor 31 zugewandt sind.

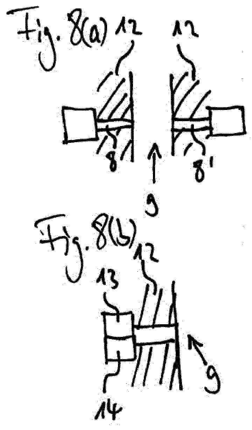

In Fign. 8(a) und (b) sind schematische Ansichten von als Sensoren zur Thrombendetektion, eines Hämatokritwerts, eines Blutzuckerspiegels bzw. eines Blutsauerstoffwerts ausgeführten faseroptischen Sensoren der Blutpumpe 2 gezeigt. Bei der Hämatokrit-, Blutzucker und Sauerstoffmessung oder Thrombuserkennung kann das transmittierte oder reflektierte Lichtspektrum ausgewertet werden. Hierbei kann eine gemessene Spektralverteilung des Lichts, beispielsweise in der Auswerteeinheit 15, mit Referenzkurven verglichen werden. Auf diese Weise können beispielsweise die Anzahl roter Blutkörperchen, die Sauerstoffsättigung, der Blutzuckergehalt und/oder der Fibringehalt gemessen werden, wobei letzterer wiederum Rückschlusse auf das Vorhandensein von Thromben erlaubt.

Lediglich in den Ausführungsbeispielen offenbarte Merkmale der verschiedenen Ausführungsformen können miteinander kombiniert und einzeln beansprucht werden.

## Patentansprüche

1. Blutpumpe (2) zum Unterstützen einer Herzfunktion, umfassend ein implantierbares Pumpengehäuse (4), **gekennzeichnet durch** einen faseroptischen Sensor (28) mit einer Lichtleitfaser (8), wobei die Lichtleitfaser (8) zumindest teilweise in einem Gehäuseteil (12) des Pumpengehäuses (4) aufgenommen ist.

2. Blutpumpe (2) nach Anspruch 1, **dadurch gekennzeichnet, dass** die Lichtleitfaser (8) zumindest teilweise in das Gehäuseteil (12) umlaufend eingebettet ist.

3. Blutpumpe (2) nach einem der Ansprüche 1 oder 2, **dadurch gekennzeichnet, dass** der faseroptische Sensor (28) ein Blutdrucksensor ist.

4. Blutpumpe (2) nach einem der Ansprüche 1 bis 3, **dadurch gekennzeichnet, dass** der faseroptische Sensor (28) ein Sensor zur Thrombendetektion ist.

5. Blutpumpe (2) nach einem der Ansprüche 1 bis 4, **dadurch gekennzeichnet, dass** eine Stirnseite (10) eines in das Gehäuseteil (12) des Pumpengehäuses (4) aufgenommenen Faserendes der Lichtleitfaser (8) nicht vollständig von dem Gehäuseteil (12) bedeckt ist.

6. Blutpumpe (2) nach Anspruch 5, **dadurch gekennzeichnet, dass** das Faserende derart in das Gehäuseteil (12) eingebettet ist, dass das Faserende und das Gehäuseteil (12) eine kunststofffreie Abdichtung ausbilden.

7. Blutpumpe (2) nach einem der Ansprüche 5 oder 6, **dadurch gekennzeichnet, dass** die Lichtleitfaser (8) mit ihrer Stirnseite (10) einem zumindest teilweise von einer innenliegenden Seite des Gehäuseteils (12) begrenzten Strömungskanal (30) zum Fördern von Blut zugewandt ist.

8. Blutpumpe (2) nach Anspruch 7, **gekennzeichnet durch** ein in dem Strömungskanal (30) angeordnetes Förderelement (31), wobei die Stirnseite (10) der Lichtleitfaser (8) an einem in Bezug zu dem Förderelement (31) stromaufwärtsliegenden Abschnitt (32) des Strömungskanals (30) angeordnet ist.

9. Blutpumpe (2) nach Anspruch 7, **gekennzeichnet durch** ein in dem Strömungskanal (30) angeordnetes Förderelement (31), wobei der faseroptische Sensor (28) eingerichtet ist, eine Position des Förderelementes (31) zu detektieren.

10. Blutpumpe (2) nach Anspruch 5 oder 6, **dadurch gekennzeichnet, dass** die Lichtleitfaser (8) mit ihrer Stirnseite (10) einer von einer außenliegenden Seite des Gehäuseteils (12) begrenzten Außenseite (29) des Pumpengehäuses (4) zugewandt ist.

11. Blutpumpe (2) nach einem der Ansprüche 1 bis 10, **dadurch gekennzeichnet, dass** das Gehäuseteil (12) Titan enthält oder aus Titan gefertigt ist.

12. Blutpumpe (2) nach einem der Ansprüche 1 bis 11, **dadurch gekennzeichnet, dass** das Gehäuseteil (12) eine Einlasskanüle (23) oder eine Kammerhälfte (25) des Pumpengehäuses (4) ist.

13. Blutpumpe (2) nach einem der vorangehenden Ansprüche, **dadurch gekennzeichnet, dass** der faseroptische Sensor (28) eine zweite Lichtleitfaser (34) aufweist, wobei die Lichtleitfasern (8, 34) derart angeordnet und ausgebildet sind, dass der faseroptische Sensor (28) ein faseroptischer Transmissionssensor ist.

14. Blutpumpe (2) nach einem der vorangehenden Ansprüche, **gekennzeichnet durch** einen zweiten faseroptischen Sensor (28), wobei der erste faseroptische Sensor (28') zur Messung eines Blutdruckes in einem von einer innenliegenden Seite des Gehäuseteils (12) begrenzten Strömungskanal (30) zum Fördern von Blut eingerichtet ist und wobei der zweite faseroptische Sensor (28, 28"') zur Messung eines Blutdruckes entweder in dem Strömungskanal (30) oder an einer Außenseite (29) des Pumpengehäuses (4) eingerichtet ist.

15. Verfahren zur Herstellung eines Pumpengehäuses (4) einer Blutpumpe (2), umfassend folgende Schritte:
- Bereitstellen einer Lichtleitfaser (8),
- Bereitstellen eines ersten Metallstücks (17),
- Anordnen der Lichtleitfaser (8) auf dem ersten Metallstück (17),
- Zumindest teilweises Überdecken der Lichtleitfaser (8) mit einem zweiten Metallstück (20) und
- Einbringen von Ultraschallenergie in das erste und/oder das zweite Metallstück (17, 20) und dadurch Verbinden des ersten und zweiten Metallstücks (17, 20) sowie Einschließen der Lichtleitfaser (8) in die Metallstücke (17, 20), derart, dass die Lichtleitfaser (8) zumindest teilweise in ein aus dem ersten Metallstück (17) und dem zweiten Metallstück (20) gebildetes Gehäuseteil (12) umlaufend eingebettet wird.
